Europäisches Patentamt

European Patent Office

Office européen des brevets

(19)

(11) **EP 0 612 404 B1**

(12) # EUROPEAN PATENT SPECIFICATION

(45) Date of publication and mention
of the grant of the patent:
**18.03.1998 Bulletin 1998/12**

(21) Application number: **93916368.9**

(22) Date of filing: **15.07.1993**

(51) Int Cl.$^6$: **G01N 1/28**

(86) International application number:
**PCT/SE93/00630**

(87) International publication number:
**WO 94/02827 (03.02.1994 Gazette 1994/04)**

(54) **INFUSING PROCESS OF INORGANIC SUBSTANCES INTO AMORPHOUS BODIES FOR ANALYSIS OF THE OXYGEN CONTENT OF THE INFUSED SAMPLES**

EINSCHMELZEN VON ANORGANISCHEN SUBSTANZEN IN AMORPHE KÖRPER ZUR ANALYSE IHRES SAUERSTOFFGEHALTS

PROCEDE D'INFUSION DE SUBSTANCES INORGANIQUES DANS DES CORPS AMORPHES PERMETTANT D'ANALYSER LA TENEUR EN OXYGENE DES ECHANTILLONS INFUSES

(84) Designated Contracting States:
**AT BE CH DE DK ES FR GB IT LI LU NL**

(30) Priority: **16.07.1992 SE 9202405**

(43) Date of publication of application:
**31.08.1994 Bulletin 1994/35**

(73) Proprietor: **JOHANSSON, Hi Kurt Erik**
**S-183 34 Täby (SE)**

(72) Inventor: **JOHANSSON, Hi Kurt Erik**
**S-183 34 Täby (SE)**

(74) Representative: **Nilsson, Karl Ingvar et al**
**STENHAGEN PATENTBYRA AB**
**P.O. Box 4630**
**116 91 Stockholm (SE)**

(56) References cited:
- **X-Ray Spectrometry, Volume 14, No. 2, 1985, D. JOSLIN et al., "Analyses of Barytes by X-Ray Fluorescence Spectrometry Using a Fusion Method for Sample Preparation", page 70, column 1, line 1 - column 2, line 31.**
- **X-Ray Spectrometry, Volume 19, 1990, K. NORRISH et al., "XRS Analysis of Sulphides by Fusion Methods", page 67, column 2, line 22 - line 41.**

**Description**

The proposed invention covers a procedure according to the preamble of claim 1 for fusing specimens in glass in such a way that the fused specimen's oxygenabsorption can be gravimetrically analysed or its oxygen content determined by other indirect means.

The fusing of specimens in glass for test preparation has been used successfully for some decades in inorganic analysis.

When using X-ray spectrometry analysis, specimens produced in this way have solved the problem of grain size effects. In optical spectrometry this type of glass is often used for testspecimens of materials containing volatile substances such as minerals formed at high pressure/temperature. The main aim of these analytical procedures has been the determination of metal or rock forming elements but not oxygen.

The technical literature describes a multitude of different ways of making glass incorporating oxidising agents with varying reactions. The use of $GeO_2$ as a glass-forming addition is previously known per se from X-RAY SPECTROMETRY; vol. 14, 1985, p, 69-73.

The main aims are to create, as far as possible, an amorphous glass and quantitatively incorporate the components of the testspecimen into the glass. Problems arise in cases where volatiles, polyoxidising oxides or sulphides are present. The treatment of specimens with volatiles is simple in cases where these can be removed at temperatures just above 100 degrees C. Where the volatiles are given off at higher temperatures the ability to determine the oxygen content is lost because one cannot determine the origin or more precisely the quantity of the absorbed oxygen from the volatiles, for example water of crystallisation. In specimens with many oxidising oxides the sum of the absorbed oxygen can be determined by this method and can in many cases lead to the determination of valences and quantitative relationships. In cases where sulphides are present the oxidation results in conversion to sulphates which are deposited in the glass. The difficulty here is that the sulphur is easily lost during the oxidation process and is considerably more unstable than the oxides. This process as defined in the appended claims suggests procedures which quantitatively bind the compounds of the particular inorganic substances in their existing valency states in order to subsequently oxidise these into higher valencies by means of a controlled oxidation process.

One trait which is frequently underestimated when analysing glasstest specimens is the change in the test specimen-fluxrelationship as a result of the specimen's oxygen absorption. For example the effect of the oxygen absorption is quite pronounced in so called Low-Dilution-Fusion (LDF) or Semilow-Dilution-Fusion (SLDF) techniques. A change in the specimen-flux relationship from 1:5 to 1.1:5 will follow from an addition of as little as 10% sulphur, corresponding to a 10% relative change. This change is much weaker, approximately 10 times less, in the oxidation of most metal oxides to a higher valency. As well as the gravimetrical determination of this change the patent applied for claims an indirect method for the determination of the specimen's oxygen content with the aid of the following algorithm.

$$BKV = P \times \{F + \Sigma R_i(Ox) + \Sigma Ox_j(Me) - \Sigma Vol_k\}^{-1};$$

where

BKV = the buffer quotient defined as the integral specimen's weight to the weight of flux
P = Weight of test specimen
F = Weight of flux
R = Reminder of added oxidising agent
$\Sigma Ox(Me)$ = The sum of the quantities of absorbed oxygen of the oxidised metaloids and/or the metal oxides
$\Sigma Vol$ = The sum of the weight of the removed volatiles
i, j, k = Indices of the weight of the included oxidising agents, whole numbers indicating the number of oxides or volatiles respectively.

As mentioned above the glass making is itself an important factor in the successful determination of oxygen absorption. Even if the example quoted concerns the use of just one type of flux including acid oxides in order to improve the glass formation and counteracting the often predominantly basic oxides, the oxygenabsorption is well defined by a number of fluxes which can form amorphous glass. The central theme of the proposed invention is characterised by the ability to quantatively fix the sulphur, the oxides present and the method of calculating the sum of teh absorbed oxygen content in accordance with the algorithm given.

Below is an example demonstrating the possibility of determining Fe(II) respectively Fe (III). The problems with two/three valencyiron occur in many cases such as in the evaluation of the state of oxidation of slags during copper production and in many mineralogical connections to take a few examples.

As has previously been pointed out when producing glass many different solid oxidising agents are used. The common factor here is that the oxidising effect takes place only a relatively high temperature 400 - 600 degrees C. In cases where the specimens contain sulphides as well as more volatile elements these must, where possible, be transformed into more stable compounds before they are melted into the glass. With ZnS it has been shown that more than 50% of the total sulphur loss occurs before the oxidation temperature of approximately 450 degrees C is reached using lithium nitrate as the oxidising agent. Thus the invention aims to oxidise or "fix" the elements or compounds at room temperature which would be volatilised during the subsequent heating. This is achieved with a basic oxidising solution. If the specimen for instance contains sulphides, these are transformed to the more stable compound lithium sulphate. It is possible to improve the containment of sulphur dioxide which may be released by the use of a "lid" of magnesium or calcium powder. Oxygen added during the oxidation and melting stage will naturally improve the oxidising conditions but is technically somewhat more complicated.

In conclusion the proposed process concerns procedures which quantitatively bind the constituent parts of the inorganic substances present in their original valency states in such a way that these are oxidised and transformed into higher valencies via controlled oxidation procedures. These are carried out in three stages within the following temperature ranges.

1. Pre-oxidation with the aid of alkaline hydroxides and hydrogen peroxide.
2. Oxidation at a higher temperature 400 - 600 degrees C approximately with the addition of a suitable quantity of lithiumnitrate.
3. Melting at approximately 1100 - 1200 degrees C with the addition of germanium dioxid in order to ensure a good glassformation which is the pre-condition for the quantitative absorption of oxygen in this process. As an acid oxide the germanium dioxide has a neutralising effect of the remaining bi-product of the added lithium nitrate, di-lithium oxide.

Example of the FeO - $Fe_2O_3$-system

In practice the most common analysis techniques used for FeO-analysis are titrometic. In these wet methods the solution's own oxidising effect can constitute a source of error during titration. The magnitude of the errors vary from a few percent (relatively) upwards depending on the composition of the testsamples. An analysis of the change in the buffer quotient (BKV), as a result of the oxygen absorption of for example FeS och FeO, it can be seen that [BKV = Flux/(Flux + test specimen)] quantitatively follows the weighed specimen. Furthermore the complete algorithm better defines the conditions when the FeO-level is determinable based on the weight of the measured oxygen. Thus besides the gravimetric determination of the oxygen the example quoted illustrates how this may be used to indirectly determine the Fe-content.

The following glass specimens for the FeO - $Fe_2O_3$--system were made according to the procedure described using specified chemicals T.M.I. < 100 ppm.

| Test specimen: | | |
|---|---|---|
| No 1 | 1.0 gram FeO | 0.0 gram $Fe_2O_3$ |
| 2 | 0.75 | 0.25 |
| 3 = Mv:A - E | 0.50 | 0.50 |
| 4 | 0.25 | 0.75 |
| 5 | 0.0 | 1.0 |

| No | After oxidation gram | After melting gram | Difference mg Melt-Oxi | Weighed $O_2$ Melt | Theoretic $O_2$ absorp |
|---|---|---|---|---|---|
| A | 6.0536 | 6.0587 | 2.0 | 58.7 | |
| B | 6.0533 | 6.0584 | 1.7 | 58.4 | |
| C | 6.0512 | 6.0564 | - 0.3 | 56.4 | |
| | | | | Mv: 55.6 | |
| D | 6.0475 | 6.0552 | - 1.5 | 55.2 | |
| E | 6.0486 | 6.0549 | - 1.8 | 54.9 | |
| | | Max error | +-2.0 | | |

(continued)

| No | After oxidation gram | After melting gram | Difference mg Melt-Oxi | Weighed $O_2$ Melt | Theoretic $O_2$ absorp |
|---|---|---|---|---|---|
| | | | | *) | **) |
| 1 | 6.0819 | 6.1021 | 20.1 | 102.0 | 111.1 |
| 2 | 6.0621 | 6.0783 | 16.2 | 78.3 | 83.3 |
| 3 | 6.0508 | 6.0567 | 5.9 | 56.7 | 55.6 |
| 4 | 6.0402 | 6.0385 | - 1.7 | 38.5 | 28.8 |
| 5 | 6.0233 | 6.0160 | - 7.7 | 16.0 | 0.0 |

*) Corrected for flux loss

**) According to the relationship $Fe_2O_3/FeO$ this corresponds to an oxygen absorption of 1.1114 mg $O_2$ / % FeO.

From these results it is shown that it is possible to determine the O-percentage or in this case the FeO-percentage with an accuracy of 3.5%. As well as the systematic flux loss there is an error with increasing FeO-percentage because of incomplete oxidation (this was later shown to be because of insufficient oxidation time). These errors can however be easily corrected.

## Claims

1. A procedure for producing amorphous test specimens, useable for gravimetric analysis of oxygen or for other indirect determination of oxygen absorption, through the oxidising and melting of the test samples intended for analysis together with flux agents and Ge $O_2$,
   **characterised by** the addition of pulverised $GeO_2$ before melting, in a porportion of 1-200 % of the test sample weight and hydrogen peroxide in a proportion of 1-500 % of the test sample weight as well as an inorganic base such as lithium- or potassium hydroxide in a proportion of 1-50 % of the test sample weight.

2. A procedure in accordance with claim 1,
   **characterised by** the further addition of an oxidising agent, usually $LiNO_3$, at a predetermined rate for the oxidation of the oxidisable elements in the test sample usually between 10-200 % of the sampleweight.

3. A procedure in accordance with claims 1 or 2,
   **characterised by** the choice of $LINO_3$ as an additional oxidising agent and the choice of glass forming lithium borates as the flux agents.

4. A procedure in accordance with claims 1 - 3,
   **characterised by** the addition of hydrogen peroxide solely or together with LiOH or any other alkaline hydroxide as a wet basic oxidising solution.

5. A procedure in accordance with claims 1 - 4,
   **characterised by** the oxidation and melting taking place in a platinum crucible.

6. A procedure in accordance with claims 1 - 5,
   **characterised by** the test specimen being exposed to heat treatment of about 105 degrees C before the melting of the test specimen.

7. A procedure in accordance with claims 1 - 6,
   **characterised by** the test specimen being exposed to an oxidising treatment at an increased temperature of about 600 degrees C before the melting of the test specimen.

8. A procedure in accordance with claims 1 - 7,
   **characterised by** the melting of the test specimen at a temperature of about 1100-1200 degrees C.

9. A procedure in accordance with claim 8,
   **characterised by** the test specimen being melted for ten minutes during which one mixing takes place.

10. A procedure in accordance with claims 7 - 9,
**characterised by** the addition of oxygen and a covering "lid" consisting of magnesium oxide powder in the proportion of 10-50 % of the test sample weight.

11. A procedure in accordance with claims 1 - 10,
**characterised by** the cooling of the test specimen to room temperature in a desiccator in order to be subsequently weighed.

12. The use of test specimens obtained using procedures in accordance with claims 1 - 11 for the analysis of oxygen or other indirect determination of oxygen and for the analysis of the oxidising elements or their elemental oxides in geological materials, inorganic environmental materials as well as sulphuric and/or oxide products from ferrous and non-ferrous production.

**Patentansprüche**

1. Verfahren zur Herstellung von amorphen Testproben, die für die gravimetrische Analyse von Sauerstoff oder für andere indirekte Bestimmungen der Sauerstoffabsorbtion verwendbar sind, durch die Oxidation und Erschmelzung von Testproben, die für die Analyse zusammen mit Flußmitteln und $GeO_2$ bestimmt sind, **gekennzeichnet durch** die Zugabe von pulverförmigem $GeO_2$ vor dem Aufschmelzen in einem Verhältnis von 1-200% des Gewichtes der Testprobe und von Wasserstoffperoxid in einem Verhältnis von 1-500% des Gewichtes der Testprobe sowie einer anorganischen Base wie beispielsweise Lithium- oder Kaliumhydroxid in einem Verhältnis von 1-50% des Gewichtes der Testprobe.

2. Verfahren nach Anspruch 1, **gekennzeichnet durch** die weitere Zugabe eines Oxidationsmittels, üblicherweise $LiNO_3$, in einem vorbestimmten Anteil für die Oxidation der oxidierbaren Elemente in der Testprobe, von üblicherweise zwischen 10-200% des Probengewichtes.

3. Verfahren nach Anspruch 1 oder 2, **gekennzeichnet durch** die Wahl von $LiNO_3$ als zusätzliches Oxidationsmittel und die Wahl von glasbildendem Lithiumborat als Flußmittel.

4. Verfahren nach einem der Ansprüche 1-3, **gekennzeichnet durch** die Zugabe von Wasserstoffperoxid allein oder zusammen mit LiOH oder einem beliebigen anderen Alkalihydroxid als wasserhaltige basische Oxidationslösung.

5. Verfahren nach einem der Ansprüche 1-4, **dadurch gekennzeichnet**, daß die Oxidation und das Aufschmelzen in einem Platintiegel erfolgen.

6. Verfahren nach einem der Ansprüche 1-5, **dadurch gekennzeichnet**, daß die Testprobe einer Wärmebehandlung bei ungefähr 105 °C ausgesetzt wird, bevor die Testprobe erschmolzen wird.

7. Verfahren nach einem der Ansprüche 1-6, **dadurch gekennzeichnet**, daß die Testprobe einer Oxidationsbehandlung bei einer erhöhten Temperatur von ungefähr 600 °C ausgesetzt wird, bevor die Testprobe erschmolzen wird.

8. Verfahren nach einem der Ansprüche 1-7, **gekennzeichnet durch** das Schmelzen der Testprobe bei einer Temperatur von ungefähr 1100-1200 °C.

9. Verfahren nach Anspruch 8, **dadurch gekennzeichnet,** daß die Testprobe für zehn Minuten aufgeschmolzen wird, währenddessen ein Mischen stattfindet.

10. Verfahren nach einem der Ansprüche 7-9, **gekennzeichnet durch** die Zugabe von Sauerstoff und eines abdeckenden "Deckels" bestehend aus pulverförmigem Magnesiumoxid in einem Verhältnis von 10-50% des Testprobengewichtes.

11. Verfahren nach einem der Ansprüche 1-10, **dadurch gekennzeichnet,** daß die Testprobe in einem Exsikkator auf Raumtemperatur abgekühlt wird, um anschließend gewogen zu werden.

12. Verwendung von Testproben, die unter Verwendung der Verfahren nach einem der Ansprüche 1-11 erhalten werden, für die Analyse von Sauerstoff oder andere indirekte Bestimmungen von Sauerstoff und für die Analyse der oxidierenden Elemente oder deren Elementoxide in geologischen Materialien, anorganischen umweltbedingten Materialien sowie von schwefelhaltigen und/oder sauerstoffhaltigen Produkten aus der Eisen- oder Nichteisen-Herstellung.

## Revendications

1. Un procédé de préparation d'éprouvettes amorphes, utilisables pour l'analyse gravimétrique de l'oxygène ou pour d'autres dosages indirects de l'absorption d'oxygène, grâce à l'oxydation et à la fusion des éprouvettes destinées à l'analyse en présence d'agents fondants et $GeO_2$, caractérisé en ce que l'on ajoute du $GeO_2$ pulvérisé avant la fusion, en une proportion de 1 à 200 % par rapport au poids de l'éprouvette et du peroxyde d'hydrogène en proportion de 1 à 500 % par rapport au poids de l'éprouvette, ainsi qu'une base inorganique telle que hydroxyde de lithium ou de potassium en une proportion de 1 à 50 % du poids de l'éprouvette.

2. Un procédé selon la revendication 1, caractérisé en ce que l'on ajoute, en plus un agent oxydant, généralement $LiNO_3$, en une proportion prédéterminée, pour l'oxydation des éléments oxydables dans l'éprouvette, généralement comprise entre 10 et 200 % du poids de l'éprouvette.

3. Un procédé selon la revendication 1 ou 2, caractérisé en ce que l'on choisit $LiNO_3$ en tant qu'agent oxydant complémentaire et en ce que l'on choisit le borate de lithium vitrifiant en tant qu'agent fondant.

4. Un procédé selon l'une quelconque des revendications 1 à 3, caractérisé en ce que l'on ajoute du peroxyde d'hydrogène seul ou en association avec LiOH ou tout autre hydroxyde alcalin, en tant que solution oxydante basique humide.

5. Un procédé selon l'une quelconque des revendications 1 à 4, caractérisé en ce que l'on effectue l'oxydation et la fusion dans un creuset en platine.

6. Un procédé selon l'une quelconque des revendications 1 à 5, caractérisé en ce que l'on expose l'éprouvette à un traitement thermique d'environ 105°C avant la fusion de l'éprouvette.

7. Un procédé selon l'une quelconque des revendications 1 à 6, caractérisé en ce que l'on expose l'éprouvette à un traitement oxydant à une température plus élevée de l'ordre de 600°C avant la fusion de l'éprouvette.

8. Un procédé selon l'une quelconque des revendications 1 à 7, caractérisé en ce que l'on effectue la fusion de l'éprouvette à une température de l'ordre de 1100-1200°C.

9. Un procédé selon la revendications 8, caractérisé en ce que l'on fond l'éprouvette pendant 10 minutes et pendant lequel on effectue un mélange.

10. Un procédé selon l'une quelconque des revendications 7 à 9, caractérisé par l'addition d'oxygène et d'une "gangue" d'enrobage consistant en poudre d'oxyde de magnésium à raison de 10 à 50 % du poids de l'éprouvette.

11. Un procédé selon l'une quelconque des revendications 1 à 10, caractérisé en ce que l'on refroidit l'éprouvette à la température ambiante dans un dessiccateur pour le peser ensuite.

12. L'utilisation d'une éprouvette obtenu par un procédé selon l'une quelconque des revendications 1 à 11, pour l'analyse de l'oxygène ou autre dosage indirect de l'oxygène et pour l'analyse des éléments oxydants ou de leurs oxydes élémentaires dans des matériaux géologiques, matériaux inorganiques de l'environnement ainsi que les produits sulfuriques et/ou oxydés de la production ferreuse et non ferreuse.